# EUROPÄISCHE PATENTANMELDUNG

(11) **EP 1 995 233 A2**
(43) Veröffentlichungstag der Anmeldung: **26.11.2008**
(21) Anmeldenummer: 08008796.8
(22) Anmeldetag: 10.05.2008
(51) Int. Cl.: C07C 68/06, B01D 3/00

(54) **Verfahren zur Herstellung von Diaryl- oder Arylalkylcarbonaten aus Dialkylcarbonaten**

(30) Priorität: 25.05.2007 DE 102007024574
(71) Anmelder: Bayer MaterialScience AG, 51368 Leverkusen (DE)
(72) Erfinder: Düx, Andre, 50321 Brühl (DE); Ooms, Pieter, Dr., 47800 Krefeld (DE); Rechner, Johann, Dr., 47906 Kempen (DE); Hallenberger, Kaspar, 51375 Leverkusen (DE); Ronge, Georg, Dr., 40549 Düsseldorf (DE); Vanden Eynde, Johan, 9052 Zwijnaarde (Gent) (BE)

(57) **Zusammenfassung**

Gegenstand der Erfindung ist ein Verfahren zur Herstellung von Diarylcarbonaten und/oder Alkylarylcarbonaten aus Dialkylcarbonaten und aromatischen Hydroxyverbindungen unter Einsatz eines oder mehrerer Zwischenkondensatoren zur Verbesserung der Energieintegration.

## Beschreibung

Gegenstand der Erfindung ist ein Verfahren zur Herstellung von Diarylcarbonaten und/oder Alkylarylcarbonaten aus Dialkylcarbonaten und aromatischen Hydroxyverbindungen unter Einsatz wenigstens einer Reaktionskolonne mit einem oder mehreren Zwischenkondensatoren zur Verbesserung der Wärmeintegration.

Die Herstellung von aromatischen und aliphatisch-aromatischen Kohlensäurestern (Carbonaten) durch Umesterung ausgehend von aliphatischen Kohlensäurestern und aromatischen Hydroxyverbindungen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv auf die Seite der aromatischen Carbonate zu verschieben, wobei nicht nur sehr aktive Katalysatoren, sondern auch geeignete Verfahrensführungen zur Anwendung gelangen müssen.

Es ist bekannt, derartige Gleichgewichtsreaktionen in Kolonnen durchzuführen und sie auf diese Weise vorteilhaft in Richtung der gewünschten Produktbildung zu verschieben (z.B. U. Block, Chem.-Ing. Techn. 49, 151 (1977); DE-OS 38 09 417; B. Schleper, B. Gutsche, J. Wnuck und L.Jeromin, Chem.-Ing.-Techn. 62, 226 (1990); Ullmans Encyclopädie der techn. Chemie, 4. Aufl., Bd. 3; S375 ff. 1973).

In den bekannten Verfahren erfolgt die Umesterung daher auch vorzugsweise kontinuierlich im Sinne einer Gegenstromumesterung in einer oder mehreren Reaktionskolonnen.

In EP-A 0 461 274 wird ein kontinuierlicher Umesterungsprozess zur Herstellung von aromatischen Carbonaten in einer oder in mehreren hintereinander geschalteten mehrstufigen Kolonnen beschrieben, wobei Dialkylcarbonate oder Alkylarylcarbonate mit Phenolen umgesetzt werden und am Kopf der Kolonnen die leichtflüchtigen Produkte, nämlich die Reaktionsalkohole und Dialkylcarbonate und am Sumpf der Kolonnen die schwersiedenden Produkte, wie z.B. Diarylcarbonate entnommen werden. Besondere Verfahrensmaßnahmen, die eine vorteilhaftere Durchführung der Umesterung unter Anpassung der Apparate und Vorgehensweisen an die oben angegebenen speziellen Probleme dieser Umesterung erlauben, sind jedoch nicht beschrieben. Insbesondere erhält der Fachmann keinen Hinweis, ob die zur Umesterung verwendete Kolonne bevorzugt mit oder ohne Verstärkungsteil ausgeführt wird.

Bei einer Ausführung mit Verstärkungsteil enthält das am Kopf der Kolonne entnommene dampfförmige Gemisch im Wesentlichen das bei der Reaktion im Überschuss eingesetzte Dialkylcarbonat und den entsprechenden, bei der Reaktion entstehenden Alkohol und die Kondensation des Gemisches erfolgt bei einer Temperatur, die unterhalb der Verdampfungstemperatur des Dialkylcarbonats bei dem in der Reaktion vorliegenden Druck liegt, mit der Folge, das die anfallende Kondensationswärme im Vergleich zur Temperatur in der Reaktionszone nur auf niedrigem Temperaturniveau abgeführt werden kann. Führt man die Umesterungskolonne ohne einen Verstärkungsteil aus, so enthält das am Kopf der Kolonne entnommene Dampfgemisch und somit auch das Destillat der Kolonne nicht unerhebliche Mengen der zur Reaktion eingesetzten aromatischen Hydroxyverbindungen und ggf. auch noch schwerer siedende Komponenten.

Bei der Rückgewinnung des im Überschuss eingesetzten Dialkylcarbonats, z.B. durch destillative Trennung vom Reaktionsalkohol führen Anteile an höher siedenden Komponenten, wie beispielsweise der aromatischen Hydroxverbindung zu einem zusätzlichen Trennproblem oder zumindest zu einem erhöhten Temperaturniveau bei der Wärmezufuhr..

DE-A 42 26 756 beschreibt ein zweistufiges Verfahren zur Herstellung von Diarylcarbonaten, bei dem in einer ersten Stufe eine aromatische Hydroxyverbindung mit einem Dialkylcarbonat in einer Gegenstromumesterung zur Reaktion gebracht wird, wobei eine Gegenstromumesterungskolonne verwendet wird, die sowohl mit als auch ohne einen Verstärkungsteil ausgeführt werden kann. Im Falle einer Anordnung mit Verstärkungsteil ist das Temperaturniveau bei der Kondensation verhältnismäßig gering, so dass die dabei anfallende Kondensationswärme nicht wirtschaftlich zum Betreiben anderer Verfahrensabschnitte verwendet werden kann. Im Falle einer Anordnung ohne Verstärkungsteil enthält das Destillat neben dem Dialkylcarbonat und dem bei Reaktion entstehenden Alkohol zudem noch einen hohen Anteil der aromatischen Hydroxyverbindung. Dies führt bei der Rückgewinnung des Dialkylcarbonats zu einem erhöhten Temperaturniveau im Bereich des Kolonnensumpfes, was die Energiezufuhr an dieser Stelle erschwert.

DE-A 42 26 755 beschreibt ein Verfahren zur Herstellung von Diarylcarbonaten in zwei energetisch und stofflich miteinander gekoppelten Reaktionskolonnen, wobei in der ersten Stufe eine aromatische Hydroxyverbindungen und ein Dialkylcarbonat, zur Reaktion gebracht werden und das dabei gebildete Alkylarylcarbonat in der zweiten Stufe entweder durch Umesterung mit der aromatischen Hydroxyverbindung oder durch Disproportionierung zum Diarylcarbonat umgesetzt wird. Problematisch ist hierbei allerdings, dass durch die stoffliche und energetische Integration des Verfahrens die Reaktionsbedingungen für die Bildung des Alkylaryl- bzw. Diarylcarbonats nicht optimal gewählt werden können, da diese durch den in beiden Schritten vorliegenden und nahezu identischen Druck festgelegt sind. Das beschriebene Verfahren liefert demnach keine Vorteile hinsichtlich Energieintegration.

Die EP-A 781 760 beschreibt ein kontinuierliches Verfahren zur Herstellung aromatischer Carbonate durch Reaktion eines Dialkylcarbonates mit einer aromatischen Hydroxyverbindung in Anwesenheit eines Katalysators, kontinuierlicher Entfernung des bei der Reaktion entstehenden aromatischen Carbonats, der alkoholischen Nebenprodukte, des Dialkylcarbonats und der aromatischen Hydroxyverbindung, wobei das Dialkylcarbonat und die aromatische Hydroxyverbindung wieder in die Reaktion zurückgeführt werden. Zwar sind die beschriebenen Verfahrensschritte effektiv bezüglich der Reaktionsführung im Sinne einer hohen Raum-ZeitAusbeute und der Aufarbeitung im Sinne einer möglichst effizienten Trennsequenz, jedoch zeigt das Verfahren keinerlei Möglichkeiten für eine energetische Integration der Reaktion und Aufarbeitungsschritte.

WO-A 2006/001256 beschreibt ein Verfahren, bei dem eine aromatische Hydroxyverbindung mit einem Dialkylcarbonat in Anwesenheit eines Katalysators umgesetzt wird sowie eine hierzu geeignete technische Vorrichtung. Auch hier werden keine Anhaltspunkte für energetische Integration gegeben.

Ohne entsprechend effiziente Energieintegration ist der Energieverbrauch der vorangehend beschriebenen Verfahren bekanntermaßen hoch, was wiederum die Vorteilhaftigkeit der phosgenfreien Herstellung von Arylcarbonaten aus ökologischer und ökonomischer Hinsicht in Frage stellt.

WO-A 2004/016577 beschreibt ein Verfahren zur Herstellung von aromatischen Carbonaten aus Dialkylcarbonat und einer aromatischen Hydroxyverbindung in Gegenwart eines Katalysators in mehreren getrennten und in Serie geschalteten Reaktionszonen einer Reaktoranordnung, wobei die anfallende Kondensationswärme bei der Kondensation des Dampfstromes der letzten Reaktionszone zur Erwärmung des in die erste Reaktionszone eingeleiteten Flüssigkeitsstromes verwendet wird. Nachteilig an diesem Verfahren ist jedoch die aufwändige Reaktoranordnung. Zudem ist die energetische Integration dieses Verfahrens verbesserungswürdig.

Es bestand demnach weiterhin Bedarf, ein Verfahren zur Herstellung von aromatischen Carbonaten, d.h. Diaryl- und/oder Alkylarylcarbonaten, vorzugsweise Diarylcarbonaten, bereitzustellen, welches die vorangehend genannten Nachteile nicht aufweist und in welchem gegenüber den vorangehend genannten bekannten Verfahren eine Energieintegration in effizienter Weise möglich ist bzw. eine verbesserte Energieintegration erreicht werden kann.

Die Aufgabe, die der Erfindung zugrunde lag, bestand demnach darin, ein Verfahren zur Herstellung von aromatischen Carbonaten, d.h. Diaryl- und/oder Alkylarylcarbonaten, vorzugsweise Diarylcarbonaten, bereitzustellen, in welchem gegenüber bekannten Verfahren eine Energieintegration in effizienter Weise möglich ist bzw. eine verbesserte Energieintegration erreicht werden kann.

Diese Aufgabe wurde erfindungsgemäß dadurch gelöst, dass durch den geeigneten Einsatz eines oder mehrerer Zwischenkondensatoren in einem Verfahren zur Herstellung von aromatischen Carbonaten, d.h. Diaryl- und/oder Alkylarylcarbonaten, aus Dialkylcarbonaten und aromatischen Hydroxyverbindungen die Energieintegration bei einfacher Verfahrensführung deutlich verbessert werden kann.

Gegenstand der vorliegenden Erfindung ist daher ein Verfahren zur Herstellung von wenigstens einem Diarylcarbonat und/oder Alkylarylcarbonat aus wenigstens einem Dialkylcarbonat und wenigstens einer aromatischen Hydroxyverbindung, wobei
(a) wenigstens ein Dialkylcarbonat in Gegenwart wenigstens eines Umesterungskatalysators mit wenigstens einer aromatischen Hydroxyverbindung in einer Umesterungskolonne enthaltend wenigstens einen Verstärkungsteil im oberen Teil der Kolonne und wenigstens eine Reaktionszone unterhalb des Verstärkungsteils, welcher mindestens zwei Sektionen aufweist, umgesetzt werden,
(b) der am Kopf der Umesterungskolonne entnommene Dampf in wenigstens einem Kondensator ganz oder teilweise kondensiert wird,
dadurch gekennzeichnet, dass wenigstens ein Verstärkungsteil der Umesterungskolonne mit wenigstens einem Zwischenkondensator ausgestattet ist und die durch Kondensation in diesem Zwischenkondensator gewonnene Kondensationswärme direkt oder indirekt wieder in das Verfahren zurückgeführt wird.

Im Rahmen der Erfindung hergestellte Diarylcarbonate sind bevorzugt solche der allgemeinen Formel (I) 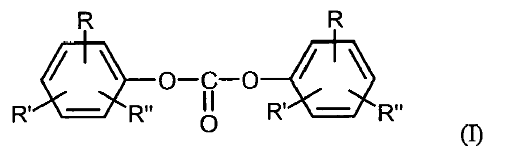 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (I) gleich oder verschieden sein können. R kann auch -COO-R'" bedeuten, wobei R"' H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenyl-carbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung bevorzugt eingesetzte Dialkylcarbonate sind solche der Formel (II) 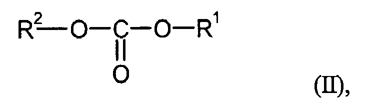 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, C₁-**C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Aryl steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Im Rahmen der Erfindung geeignete aromatische Hydroxyverbindungen sind bevorzugt solche der allgemeinen Formel (III) 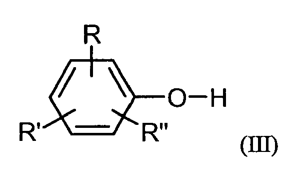 worin R, R' und R" unabhängig voneinander die für die allgemeine Formel (I) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-isoOctylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte Diarylverbindungen sind Phenol, 4-tert-Butylphenol, Biphenyl-4-ol und 4-(1-Methyl-1-phenylethyl)-phenol.

Besonders bevorzugt ist Phenol.

Im Rahmen der Erfindung hergestellte Alkylarylcarbonate sind bevorzugt solche der allgemeinen Formel (IV) 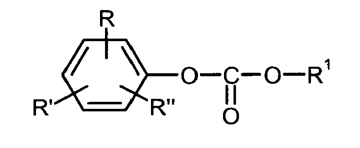 worin R, R' und R" die für die allgemeine Formel (I) und R¹ die für die allgemeine Formel (II) genannte Bedeutung haben können.

Bevorzugte Alkylarylcarbonate sind Methyl-phenyl-carbonat, Ethyl-phenyl-carbonat, Propyl-phenyl-carbonat, Butylphenyl-carbonat und Hexyl-phenyl-carbonat, Methyl-(o-kresyl)-carbonat, Methyl-(p-kresyl)-carbonat, Ethyl-(o-kresyl)-carbonat, Ethyl-(p-kresyl)-carbonat, Methyl- oder Ethyl-(p-chlorphenyl)-carbonat. Besonders bevorzugte Alkylarylcarbonate sind Methyl-phenyl-carbonat und Ethyl-phenyl-carbonat. Ganz besonders bevorzugt ist Methyl-phenyl-carbonat.

Sowohl die für das erfindungsgemäße Verfahren geeigneten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

Im erfindungsgemäßen Verfahren werden die aromatische(n) Hydroxyverbindung(en) und das oder die Dialkylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 eingesetzt. Dabei berücksichtigt das angegebene Molverhältnis nicht die Rückführung von aromatischer Hydroxyverbindung oder Dialkylcarbonat in die Umesterungskolonne über einen oder mehrere Kopfkondensator(en) (vgl. unter (b)) oder einen oder mehrere etwaig vorhandene(n) Sumpfverdampfer.

Das erfindungsgemäße Verfahren wird in einer Umesterungskolonne durchgeführt. In bevorzugten Ausführungsformen des erfindungsgemäßen Verfahrens kann der am Sumpf dieser Umesterungskolonne entnommene Flüssigkeitsstrom - gegebenenfalls nach Aufkonzentrierung - in einem oder mehreren weiteren Schritten einer Disproportionierung, einer weiteren Umesterung und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die Umesterungskolonne enthält wenigstens einen Verstärkungsteil im oberen Teil der Kolonne und wenigstens eine Reaktionszone unterhalb des Verstärkungsteils, welcher mindestens zwei Sektionen aufweist, wobei wenigstens ein Verstärkungsteil der Umesterungskolonne mit wenigstens einem Zwischenkondensator ausgestattet ist. Jede der zwei Sektionen weist unabhängig voneinander bevorzugt jeweils 0 bis 20, bevorzugt 0,1 bis 20 theoretische Stufen auf. Der Zwischenkondensator ist vorzugsweise zwischen den beiden Sektionen des Verstärkungsteils angebracht. In diesem Fall wird der Verstärkungsteil in einen oberen und einen unteren Verstärkungsteil geteilt.

Die Umesterungskolonne wird bevorzugt im Gegenstrom betrieben, wobei vorzugsweise in wenigstens einer Reaktionszone dieser Kolonne die aromatische Hydroxyverbindung flüssig vom Kopf zum Sumpf geführt und das Dialkylcarbonat gasförmig diesem flüssigen Strom entgegengeführt wird. Dabei wird die Umesterungskolonne vorzugsweise so betrieben, dass man wenigstens einer Reaktionszone, bevorzugt in das obere Drittel der Reaktionszone, einen oder mehrere, die aromatische Hydroxyverbindung und gegebenenfalls gelösten Umesterungskatalysator enthaltende Ströme, bevorzugt mit der an dieser Stelle der Kolonne herrschenden Temperatur, flüssig oder mit nur geringem Gasanteil eindosiert, wobei der Gasanteil bevorzugt weniger als 20 Gew.-% beträgt. Zudem werden einer oder mehrere, das Dialkylcarbonat enthaltende Ströme in die Reaktionszone, bevorzugt im unteren Drittel dieser Reaktionszone, gleitet, wobei die Zudosierung vorzugsweise gasförmig oder überhitzt erfolgt. In bevorzugten Ausführungsformen kann die Überhitzung des Dampfstroms 0 bis 50 °C betragen. Des Weiteren richtet sich die Taupunktstemperatur bevorzugt nach dem in der Reaktionszone an der Zudosierstelle des jeweiligen Dialkylcarbonat enthaltenden Stromes vorliegenden Druck.

Nach Passieren der Reaktionszone(n) wird der Reaktionsalkohol, nach Durchlaufen des oder der Verstärkungsteile, am Kopf der Umesterungskolonne entnommen. Beim Reaktionsalkohol handelt es sich im Rahmen der Erfindung um den bei der Umesterung frei werdenden Alkohol, bevorzugt R¹-OH bzw. R²-OH, wobei R¹ und R² die für die allgemeine Formel (II) genannte Bedeutung haben. Der am Kopf der Umesterungskolonne entnommene Strom enthält im Allgemeinen zusätzlich zum Reaktionsalkohol noch überschüssiges oder nicht umgesetztes Dialkylcarbonat. Aufgrund des oder der vorhandenen Verstärkungsteil(e) enthält dieser Strom nur geringe Mengen an höher siedenden Komponenten, wie z.B. der aromatischen Hydroxyverbindung. Der Verstärkungsteil dient zur Abtrennung der in der Reaktionszone mitverdampften höher siedenden Komponenten, wie z.B. der aromatischen Hydroxyverbindung oder Alkylarylcarbonat von den leichtsiedenden Reaktionsalkoholen oder Dialkylcarbonaten. Dies hat den Vorteil, dass die Trennung der Reaktionsalkohole von den Dialkylcarbonaten bei einem niedrigen Temperaturniveau durchgeführt werden kann.

Die Umesterungskolonne wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert (vgl. unter (b)) und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

In bevorzugten Ausführungsformen weist die Umesterungskolonne unterhalb einer Reaktionszone wenigstens einen Abtriebsteil auf.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Bevorzugt ist der Verstärkungsteil der Umesterungskolonne, der mit wenigstens einem Zwischenkondensator ausgestattet ist, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden.

Der oder die Verstärkungsteil(e) mit wenigstens einem Zwischenkondensator können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung der aromatischen Hydroxyverbindung stattfindet. Das aus dieser unteren Sektion bzw. gegebenenfalls dem unteren Verstärkungsteil kommende dampfförmige Gemisch wird in einen Zwischenkondensator geleitet, wo dieses teilweise auskondensiert und das anfallende Kondensat am oberen Ende der unteren Sektion des Verstärkungsteils bzw. gegebenenfalls dem unteren Verstärkungsteil zugeführt wird.

In einer weiteren bevorzugten Ausführungsform des erfindungsgemäßen Verfahrens wird der Zwischenkondensator nicht in die Umesterungskolonne integriert sondern als separater Zwischenkondensator außerhalb der Umesterungskolonne ausgeführt.

In einer weiteren bevorzugten Ausführungsform des erfindungsgemäßen Verfahrens werden Zwischenkondensator und die obere Sektion des Verstärkungsteils nicht in die Umesterungskolonne integriert sondern separat außerhalb der Umesterungskolonne untergebracht.

Unterhalb der Reaktionszone und eines gegebenenfalls vorhandenen Abtriebsteils wird ein Gemisch enthaltend Alkylarylcarbonat, überschüssigem oder nichtumgesetztem Phenol, Diarylcarbonat, Umesterungskatalysatoren, Dialkylcarbonat, Reaktionsalkohol und bei der Reaktion entstehende oder bereits in den Edukten enthaltene schwersiedende Verbindungen erhalten.. Bei Verwendung eines Abtriebsteils wird der Gehalt an leichtsiedenden Verbindungen, wie beispielsweise Dialkylcarbonat und Reaktionsalkohol reduziert, wobei in Anwesenheit des Umesterungskatalysators unter Umständen weiteres Alkylarylcarbonat und/oder Diarylcarbonat gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 150 bis 240°C. In bevorzugten Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0.5 bis 20 bar, besonders bevorzugt von 0.8 bis 15 bar, ganz besonders bevorzugt von 0.9 bis 10 bar. Bei den vorangehend und im Folgenden aufgeführten Druckangaben handelt es sich - sofern nichts anderes explizit erwähnt - um absolute Druckangaben.

Für die in der Umesterungskolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie z. B. Hydride, Oxide, Hydroxide, Alkoholate, Amide und andere Salze von Alkali- und Erdalkalimetallen, wie von Lithium, Natrium, Kalium, Rubidium, Caesium, Magnesium und Calcium, bevorzugt Lithium, Natrium, Kalium, Magnesium und Calcium und besonders bevorzugt Lithium, Natrium und Kalium (vgl. z.B. US 3 642 858, US 3 803 201 oder EP-A 1082). Salze der Alkali- und Erdalkalimetalle können auch solche von organischen oder anorganischen Säuren sein, wie von Essigsäure, Propionsäure, Buttersäure, Benzoesäure, Stearinsäure, Kohlensäure (Carbonate oder Hydrogencarbonate), Phosphorsäure, Blausäure, Rhodanwasserstoffsäure, Borsäure, Zinnsäure, C₁₄-Stannonsäuren oder Antimonsäure. Bevorzugt kommen als Verbindungen der Alkali- und Erdalkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Die genannten Alkali- oder Erdalkalimetallverbindungen werden bevorzugt in Mengen von 0,001 bis 2 Gew.-%, bevorzugt von 0,005 bis 0,9 Gew.-% und besonders bevorzugt von 0,01 bis 0,5 Gew.-%, bezogen auf das Gewicht des umzusetzenden Reaktionsgemischs, eingesetzt.

Weitere erfindungsgemäß einsetzbare Katalysatoren sind Metallverbindungen wie AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)_{4-X}-Sn(Y)_{X}, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹ Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenylstannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiC1, Pb(OCO-CH₃)₂ · 2PPh3 in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den erfindungsgemäßen Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die Umesterungskolonne über eingebracht. Alternativ kann der Katalysator auch separat beispielsweise im Reaktionsalkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der Umesterungskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) als auch mit dem gasförmig eindosierten, Dialkylcarbonat enthaltenden Strom eingebracht werden. Insbesondere im Bereich der Reaktionszone(n) kann eine Zufuhr von Wärme auf diese Weise erfolgen. Vorzugsweise wird diese Wärme im Bereich der Reaktionszone(n) ganz oder teilweise mittels Verdampfer oder beheizbarer Kolonnenböden zugeführt. Besonders vorteilhaft ist es, die für die Reaktion in der Umesterungskolonne erforderliche Energie wenigstens teilweise sowohl mit dem flüssigen Strom enthaltend die aromatische(n) Hydroxyverbindung(en) als auch mit dem gasförmig eindosierten, Dialkylcarbonat enthaltenden Strom in die Umesterungskolonne und zusätzlich durch innen- und/oder außen liegende Wärmetauscher einzubringen.

Die durch Kondensation in dem oder den Zwischenkondensator(en) gewonnene Kondensationswärme wird erfindungsgemäß direkt oder indirekt wieder in das Verfahren zurückgeführt. Unter direkter Rückführung der Kondensationswärme in das Verfahren ist im Rahmen der Erfindung zu verstehen, dass diese Kondensationswärme ohne zwischengeschaltetes Heizmedium in das Verfahren zurückgeführt wird, z.B. zur Erwärmung entweder eines der Umesterungskolonne oder einer gegebenenfalls zweiten Kolonne zugeführten Stromes oder zur Erwärmung eines oder mehrerer Kolonnenabschnitte. Dies kann beispielsweise in einem Wärmetauscher erfolgen. Vorzugsweise ist dabei entweder ein solcher Wärmeaustauscher mit dem Zwischenkondensator kombiniert. Unter indirekter Rückführung der Kondensationswärme in das Verfahren ist im Rahmen der Erfindung zu verstehen, dass mit der gewonnen Kondensationswärme zunächst ein Heizmedium erzeugt wird, welches zur Rückführung der Kondensationswärme in das Verfahren dient. Mit diesem Heizmedium kann beispielsweise ein der Umesterungskolonne oder einer gegebenenfalls zweiten Kolonne zugeführter Strom oder einer oder mehrere Kolonnenabschnitte erwärmt werden. Als Heizmedium kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl^{™}, Marlotherm^{®}). Besonders bevorzugte Heizmedien sind Wasser oder Wasserdampf. In einer besonders bevorzugten Ausführungsform des erfindungsgemäßen Verfahrens wird die im Zwischenkondensator gewonnenen Kondensationswärme direkt oder indirekt, ganz oder teilweise zur Verdampfung des in die Umesterungskolonne eingeleiteten Dialkylcarbonats verwendet.

Das erfindungsgemäße Verfahren kann kontinuierlich oder diskontinuierlich durchgeführt werden. Bevorzugt ist eine kontinuierliche Fahrweise.

Durch die Verwendung eines oder mehrerer Zwischenkondensator(en) bei der Umesterung in der Umesterungskolonne kann die bei der Kondensation gewonnene Kondensationswärme auf einem deutlich höheren Temperaturniveau abgeführt werden und daher effizienter genutzt werden. Dadurch können Heiz- und Kühlleistung in gleichem Maße reduziert werden. Ein wesentlicher Vorteil des erfindungsgemäßen Verfahrens gegenüber den Verfahren gemäß Stand der Technik liegt daher in der deutlichen Reduzierung des Energieverbrauchs bei der Herstellung von Diarylcarbonaten bzw. Alkylarylcarbonaten. Gleichzeitig kann das Verfahren mit einfachem apparativem Aufwand durchgeführt werden, da aufgrund der Verwendung von Kolonnenanordnungen keine komplizierte Reaktoranordnung mit mehreren getrennten und in Serie geschalteten Reaktionszonen erforderlich ist.

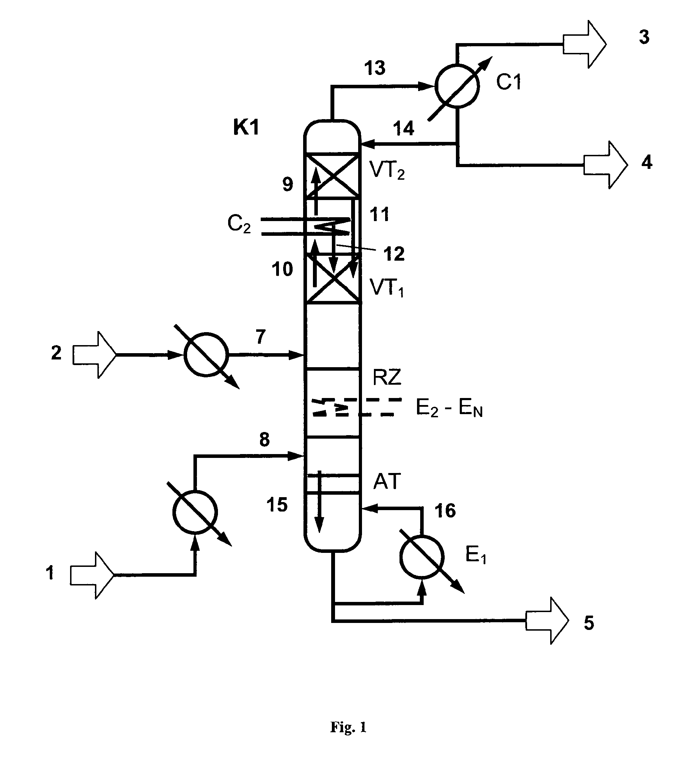

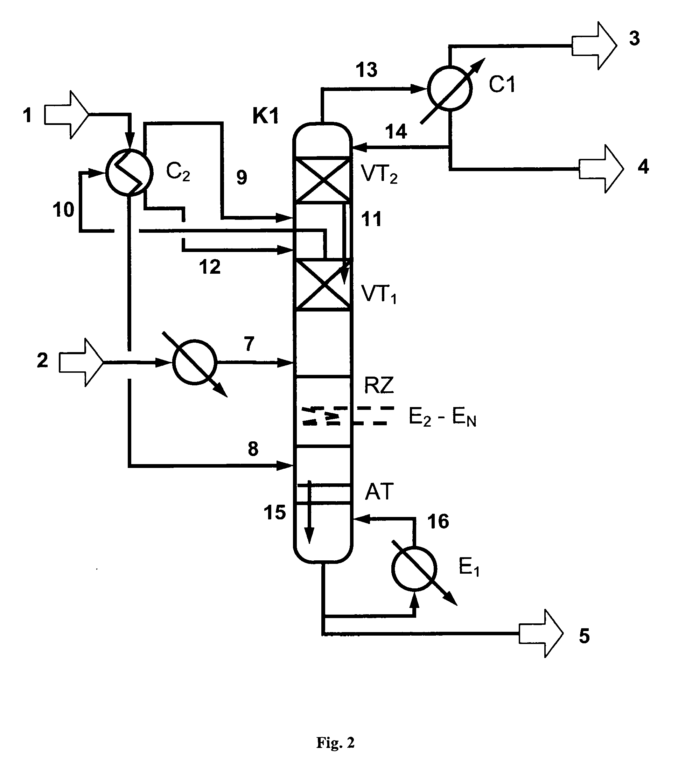

Das erfindungsgemäße Verfahren wird ausschnittsweise mit Hilfe der Fig. 1 bis 3 beispielhaft erläutert. Die Fig. 1 bis 3 zeigen das erfindungsgemäße Verfahrens ohne etwaige nachfolgende Schritte wie Disproportionierung, weitere Umesterung oder Reinigung in etwaigen weiteren Kolonnen. Eine Ausführungsform der Rückführung der gewonnenen Kondensationswärme ist lediglich in Fig. 2 gezeigt.

Fig. 1 beschreibt eine Reaktivrektifikation in der Umesterungskolonne mit Zwischenkondensator im Allgemeinen.

Fig. 2 beschreibt eine Reaktivrektifikation mit externer Anordnung des Zwischenkondensators und Kombination mit der Verdampfung des Dialkylcarbonats zur Rückführung der anfallenden Kondensationswärme.

Fig. 3 beschreibt eine Reaktivrektifikation mit externer Anordnung des Zwischenkondensator und externer Anordnung des oberen Verstärkungsteils in einer separaten Kolonne.

Die Figuren dienen der beispielhaften Erläuterung der Erfindung und sind nicht als Beschränkung aufzufassen.

In den Fig. 1 bis 3 bedeuten
- K1: Umesterungskolonne
- K2: Kolonne mit separatem Verstärkungsteil
- C1: Kondensator
- C₂: Zwischenkondensator
- VT₁: unterer Verstärkungsteil
- VT₂: oberer Verstärkungsteil
- RZ: Reaktionszone
- AT: Abtriebsteil
- E₁: Verdampfer für Sumpfprodukt
- E₂-E_{N}: Zwischenverdampfer in der Reaktionszone

Weiterhin sind in den Fig. 1 bis 3 die folgenden Stoffströme benannt
- 1: Strom enthaltend Dialkylcarbonat(e) (flüssig)
- 2: Strom enthaltend aromatische Hydroxyverbindung(en)
- 3: Restliches Dampfgemisch nach der Kondensation in Kondensator C1
- 4: Destillat der Umesterungskolonne nach der Kondensation in Kondensator C 1
- 5: Sumpfprodukt der Umesterungskolonne nach Aufkonzentierung durch Sumpfverdampfer E₁
- 7: Strom mit aromatischer Hydroxyverbindungen nach Erhitzung
- 8: Strom mit Dialkylcarbonat nach Verdampfung (und ggf. Überhitzung)
- 9: Dampfförmiges Gemisch nach Zwischenkondensator C₂
- 10: Dampfförmiges Gemisch zum Zwischenkondensator C₂
- 11: Ablaufende Flüssigkeit vom oberen Verstärkungsteil VT₂
- 12: Kondensat des Zwischenkondensators C₂
- 12a: Flüssigkeit zum unteren Verstärkungsteil VT₁
- 13: Dampfförmiges Gemisch zum Kondensator C1
- 14: Rücklauf zum oberen Verstärkungsteil VT₂
- 15: Ablauf Flüssigkeitsgemisch aus Abtriebsteil AT
- 16: Dampf-/Flüssigkeitsgemisch am Austritt des Verdampfers E₁

Fig. 1 zeigt eine Umesterungskolonne K1, in die die beiden Eduktströme, d.h. ein die aromatische Hydroxyverbindung enthaltender Strom 2 und ein das Dialkylcarbonat enthaltender Strom 1 im Bereich einer Reaktionszone RZ im Sinne eine Gegenstromumesterung gegeneinander geführt und zu Alkylarylcarbonaten und geringen Anteilen Diarlycarbonaten umgesetzt werden.

Der das Dialkylcarbonat enthaltende Strom 1 kann - insbesondere bei kontinuierlich geführten Verfahren - neben dem Dialkylcarbonat auch Teile der aromatischen Hydroxyverbindung, die bei der Reaktion anfallende aliphatische Hydroxyverbindung R¹-OH und/oder R²-OH (Reaktionsalkohol), sehr geringe Mengen des bei der Umesterung anfallenden Alkylarylcarbonats und/oder Diarylcarbonats und bei der Reaktion entstehende unerwünschte Nebenkomponenten enthalten. Der das Dialkylcarbonat enthaltende Strom 1 kann beispielsweise 0 bis 5 Gew.-%, bevorzugt 0.05 bis 3 Gew: % und besonders bevorzugt 0.05 bis 2 Gew: % des Reaktionsalkohols, 0 bis 40 Gew.-%, bevorzugt 0 bis 10 Gew.-%, besonders bevorzugt 0 bis 5 Gew.-% der aromatischen Hydroxyverbindung, 0 bis 5 Gew.-% Alkylarylcarbonat, 0 bis 5 Gew.-% Diarylcarbonat und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dialkylcarbonat enthaltenden Stromes, aufweisen. Vorzugsweise enthält der das Dialkylcarbonat enthaltende Strom 1 50 bis 100 Gew.-% Dialkylcarbonat, bezogen auf das Gesamtgewicht des Dialkylcarbonat enthaltenden Stromes, wobei sich die Anteile der einzelnen vorangehend genannten Komponenten zu 100 Gew.-% addieren. Der die aromatische Hydroxyverbindung enthaltende Strom 2 kann - insbesondere bei kontinuierlich geführten Verfahren - neben der aromatischen Hydroxyverbindung auch Teile des Dialkylcarbonats, das bei der Umesterung anfallende Alkylarylcarbonat und/oder Diarylcarbonat, in sehr geringen Mengen den Reaktionsalkohol und bei der Reaktion entstehende unerwünschte Nebenprodukte enthalten. Beispielsweise kann der Gehalt des Dialkylcarbonats 0 bis 50 Gew.-%, der Gehalt des Reaktionsalkohols 0 bis 10 Gew.-%, bevorzugt 0 bis 5 Gew.-%, der Gehalt des Alkylarylcarbonats und des Diarylcarbonats jeweils 0 bis 10 Gew.-%, bevorzugt 0 bis 5 Gew.-% und der Gehalt der unerwünschten Nebenprodukte 0 bis 5 Gew.-%, bevorzugt 0 bis 1 Gew.-%, jeweils bezogen auf das Gesamtgewicht des die aromatische Hydroxyverbindung enthaltenden Stromes, betragen. Mit dem die aromatische Hydroxyverbindung enthaltenden Strom 2 kann zudem der Katalysator in die Umesterungskolonne eingespeist werden. In diesem Fall beträgt der Gehalt an Katalysator bevorzugt 0 bis 5 Gew.-%, bezogen auf das Gesamtgewicht des die aromatische Hydroxyverbindung enthaltenden Stromes. Vorzugsweise enthält der die aromatische Hydroxyverbindung enthaltende Strom 2 50 bis 100 Gew.-% aromatische Hydroxyverbindung, bezogen auf das Gesamtgewicht des die aromatische Hydroxyverbindung enthaltenden Stromes, wobei sich die Anteile der einzelnen vorangehend genannten Komponenten zu 100 Gew.-% addieren.

Der das Dialkylcarbonat enthaltende Strom 1 wird vor Einleiten in die Kolonne K1 verdampft und ggf. überhitzt. Der die aromatische Hydroxyverbindung enthaltende Strom 2 wird vor Einleiten in die Kolonne K1 erhitzt. Die Eduktströme 7 und 8, jeweils nach Verdampfung und gegebenenfalls Überhitzung bzw. nach Erhitzen, werden in der Reaktionszone RZ im Gegenstrom zueinander geführt, d.h. der die aromatische Hydroxyverbindung enthaltende Strom 7 wird am oberen Ende der Reaktionszone RZ in erhitzter, flüssiger Form und der das Dialkylcarbonat enthaltende Strom 8 wird am unteren Ende der Reaktionszone gasförmig oder gegebenenfalls leicht überhitzt zugeführt. Die bei der Reaktion anfallende aliphatische Hydroxyverbindung R¹-OH und/oder R²-OH wird zusammen mit noch nicht umgesetzten Dialkylcarbonat am Kopf der Kolonne dampfförmig abgezogen (13) und das schwererflüchtige Alkylarylcarbonat zusammen mit noch nicht umgesetzten Mengen der aromatischen Hydroxyverbindung, Diarylcarbonat und gegebenenfalls weiteren schwerflüchtigen Verbindungen als flüssiger Strom am Fuß der Kolonne K1 entnommen (5). Die zur Einstellung des gewünschten Temperaturprofils erforderliche Energie kann unter Anderem am Sumpf der Kolonne durch einen oder mehrere Verdampfer E₁ erfolgen. Hierzu wird das aus dem Abtriebsteil, bzw. bei nicht vorhandenem Abtriebsteil aus der Reaktionszone, ablaufende flüssige Gemisch (15) teilweise verdampft. Je nach Verdampferausführung erhält man am Austritt des Verdampfers nur Dampf oder ein Dampf-Flüssigkeitsgemisch (Strom 16). Der im Strom 16 enthaltene Dampf wird dem Abtriebsteil (AT) von unten, oder falls kein Abtriebsteil vorhanden ist, der Reaktionszone von unten zugeführt. Im Bereich der Reaktionszone kann durch zusätzliche Zwischenverdampfer E₂-E_{N} Wärme zugeführt werden. Im zwischen Reaktionszone RZ und Verdampfer E₁ angebrachten Abtriebsteil AT erfolgt eine Aufkonzentrierung des gebildeten Alkylarylcarbonats und des Diarylcarbonats, wobei bereits in diesem Teil der Kolonne K1 durch die Verarmung an Dialkylcarbonat die Disproportionierungsreaktion von Alkylarylcarbonat zu Diarylcarbonat in verstärktem Maße einsetzt.

In einem zwischen Kondensator C1 und Reaktionszone RZ befindlichen Verstärkungsteil erfolgt die Aufkonzentrierung der bei der Reaktion gebildeten aliphatischen Hydroxyverbindung und des überschüssigen Dialkylcarbonats. Dabei soll ein Gehalt an aromatischer Hydroxyverbindung(en) im Destillat 4 von 0 bis 40 Gew.-% bevorzugt 0 bis 10 Gew.-%, besonders bevorzugt von 0 bis 5 Gew.-%, bezogen auf Gesamtgewicht des Destillats 4, eingestellt werden. Der Verstärkungsteil ist in mindestens zwei Sektionen, den oberen und den unteren Verstärkungsteil, unterteilt, wobei sich zwischen dem oberen Verstärkungsteil VT₂ und dem unteren Verstärkungsteil VT₁ ein Zwischenkondensator C₂ befindet. Dieser Zwischenkondensator C₂ kondensiert einen Teil der aus dem unteren Verstärkungsteil VT₁ aufsteigenden Dämpfe 10. Das in den Zwischenkondensator C₂ eintretende dampfförmige Gemisch 10 enthält vorzugsweise 10 bis 70 Gew.-% an aromatischer Hydroxyverbindung. Die Kondensationstemperatur im Zwischenkondensator C₂ ist daher aufgrund verhältnismäßig hoher Mengen an aromatischer Hydroxyverbindung deutlich höher im Vergleich zur Kondensationstemperatur im Kopfkondensator C1. Je nach Betriebsdruck und Lage des Konzentrationsprofils kann die Kondensationstemperatur im Zwischenkondensator bevorzugt im Bereich von 100 bis 300°C, besonders bevorzugt von 120 bis 250°C, ganz besonders bevorzugt von 150 bis 240°C und im Kopfkondensator bevorzugt im Bereich von 0 bis 250°C, besonders bevorzugt von 40 bis 200°C liegen. Das im Zwischenkondensator C₂ anfallende Kondensat 12 sowie die aus dem darüber liegenden oberen Verstärkungsteil VT₂ ablaufende Flüssigkeit 11 wird auf den unteren Verstärkungsteil VT₁ geleitet. Das dampfförmige Gemisch nach dem Zwischenkondensator gelangt in den oberen Verstärkungsteil VT₂. Der aus dem oberen Verstärkungsteil VT₂ kommende Dampf 13 wird im Kondensator C1 weitestgehend kondensiert, wobei das Kondensat teilweise wieder als Rücklauf dem oberen Verstärkungsteil VT₂ zugeführt wird (14) und teilweise als Destillatstrom 4 entnommen wird. Der Destillatstrom 4 enthält im wesentlichen das im Überschuss eingesetzte Dialkylcarbonat und die entsprechende bei der Reaktion entstehende aliphatische Hydroxyverbindung R¹-OH und/oder R²-OH und gegebenenfalls geringe Mengen der aromatischen Hydroxyverbindung. Das restliche Dampfgemisch aus dem Kondensator C1 wird als Dampfstrom 3 entnommen.

Die im Zwischenkondensator C₂ frei werdende Kondensationswärme kann wie vorangehend für das erfindungsgemäße Verfahren beschrieben direkt oder indirekt wieder in das Verfahren zurückgeführt werden (In Fig. 1 nicht gezeigt).

Fig. 2 zeigt eine besonders bevorzugte Ausführungsform des erfindungsgemäßen Verfahrens, bei der der Zwischenkondensator separat außerhalb der Kolonne ausgeführt ist und die bei der Kondensation des Stromes 10 im Zwischenkondensator C₂ anfallende Kondensationswärme zur Verdampfung des bei der Gegenstromumesterung in der Umesterungskolonne K1 eingesetzten, Dialkylcarbonat enhaltenden Stromes 1 verwendet wird. Hierzu wird der das Dialkylcarbonat enhaltende Strom 1 einer Verdampfungseinheit C₂ zugeführt, ganz oder teilweise verdampft und gegebenenfalls noch überhitzt. Das dampfförmige Gemisch 10 des unteren Verstärkungsteils VT₁ wird zum Zwischenkondensator C₂ geleitet, wo dieses teilweise kondensiert. Das dabei anfallende Kondensat 12 wird wieder dem unteren Verstärkungsteil VT₁ zugeführt, die nicht kondensierten Dämpfe werden in den oberen Verstärkungsteil VT₂ geleitet. Ansonsten entspricht das in Fig. 2 gezeigte Verfahren dem in Fig. 1 dargestellten. Die vorangehenden Erläuterungen für Fig. 1 gelten daher analog.

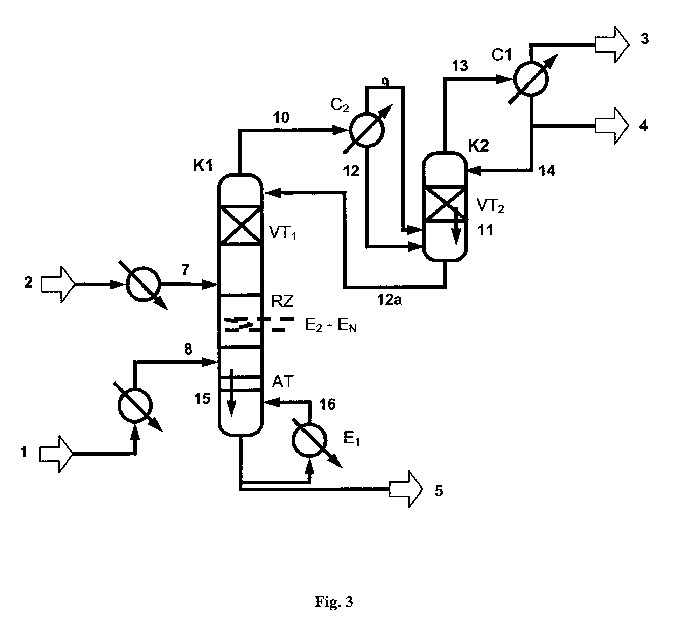

Fig. 3 zeigt eine weitere besonders bevorzugte Ausführungsform des erfindungsgemäßen Verfahrens, bei der der Zwischenkondensator C₂ und der obere Verstärkungsteil VT₂ nicht in die Umesterungskolonne K1 integriert sondern separat außerhalb der Kolonne K1 ausgeführt sind. Dabei wird das aus dem unteren Verstärkungsteil VT₁ kommende dampfförmige Gemisch 10 dem Zwischenkondensator C₂ zugeführt und dort teilweise kondensiert. Der restliche Dampf wird dem oberen Verstärkungsteil VT₂, der in einer separaten Kolonne K2 untergebracht ist, zugeführt. Die aus dem oberen Verstärkungsteil VT₂ kommende Flüssigkeit 11 wird zusammen mit dem Kondensat 12 aus dem Zwischenkondensator C₂ dem unteren Verstärkungssteil zugeführt (12a). Der aus dem oberen Verstärkungsteil VT₂ kommende Dampf 13 wird weitestgehend kondensiert, wobei das Kondensat teilweise wieder als Rücklauf dem oberen Verstärkungsteil VT₂ zugeführt wird (14) und teilweise als Destillatstrom 4 entnommen wird. Der Destillatstrom 4 enthält im wesentlichen das im Überschuss eingesetzte Dialkylcarbonat und die entsprechende bei der Reaktion entstehenden aliphatische Hydroxyverbindung R¹-OH und/oder R²-OH und gegebenenfalls geringe Mengen der aromatischen Hydroxyverbindung. Das restliche Dampfgemisch aus dem Kondensator C1 wird als Dampfstrom 3 entnommen. Ansonsten entspricht das in Fig. 3 gezeigte Verfahren dem in Fig. 1 dargestellten. Die vorangehenden Erläuterungen für Fig. 1 gelten daher analog.

Für das erfindungsgemäße Verfahren geeignete Umesterungskolonnen enthaltend wenigstens einen Zwischenkondensator sind in der Literatur bisher nicht beschrieben. Daher ist weiterhin Gegenstand der vorliegenden Erfindung eine Kolonne für die Umsetzung von Dialkylcarbonaten mit aromatischen Hydroxyverbindungen in Gegenwart wenigstens eines Umesterungskatalysators zur Herstellung von Diarylcarbonaten und/oder Alkylarylcarbonaten enthaltend
- wenigstens einen Einlass für das oder die Dialkylcarbonat(e) und wenigstens einen Einlass für das oder die aromatische Hydroxyverbindung(en)
- wenigstens einen Auslass für das gasförmige Kopfprodukt im oberen Teil der Kolonne und wenigstens einen Auslass für das flüssige Sumpfprodukt im unteren Teil der Kolonne
- wenigstens einen Verstärkungsteil im oberen Teil der Kolonne und wenigstens eine Reaktionszone unterhalb eines Verstärkungsteils, welcher mindestens zwei Sektionen aufweist.
**dadurch gekennzeichnet**, dass wenigstens ein Verstärkungsteil mit wenigstens einem Zwischenkondensator ausgestattet ist.

Bevorzugt weist die erfindungsgemäße Umesterungskolonne im Bereich der Reaktionszone einen Durchmesser D_{RZ} von 400 bis 20000 mm und die Reaktionszone eine Länge L_{RZ} von 5000 bis 60000 mm auf. Weiterhin bevorzugt weist die erfindungsgemäße Umesterungskolonne in der Reaktionzone eine Anzahl theoretischer Stufen n_{Rz} von 5 bis 50 auf.

Die Reaktionszone kann zudem eine Anzahl Zwischenerhitzer n_{E} aufweisen. Dass Verhältnis der Anzahl Zwischenerhitzer n_{E} in der Reaktionszone zur Anzahl theoretischer Stufen n_{RZ} beträgt dabei bevorzugt 0 bis 2.

Weiterhin bevorzugt weist die erfindungsgemäße Umesterungskolonne im Bereich des gegebenenfalls vorhandenen Abtriebsteils einen Durchmesser D_{AT} von 100 bis 20000 mm und der Abtriebsteil eine Länge L_{AT} von 100 bis 20000 mm auf. Weiterhin bevorzugt weist die erfindungsgemäße Umesterungskolonne im Abtriebsteil eine Anzahl theoretischer Stufen n_{AT} von 0 bis 20 auf.

Bevorzugt weist das Verstärkungsteil der erfindungsgemäßen Umesterungskolonne einen Durchmesser D_{VT} von 200 bis 10000 mm und der gesamte Bereich des Verstärkungsteils - einschließlich gegebenenfalls vorhandenem oberen und unterem Verstärkungsteil und Zwischenkondensator - eine Länge L_{VT} von 100 bis 30000 mm , bevorzugt von 500 bis 30000mm auf. Weiterhin bevorzugt weist Verstärkungsteil insgesamt eine Anzahl theoretischer Stufen n_{VT} von 2 bis 20 auf.

In bevorzugten Ausführungsformen weist der untere Verstärkungsteil der erfindungsgemäßen Umesterungskolonne einen Durchmesser D_{VT1} von 200 bis 10000 mm und eine Länge L_{VT1} von 200 bis 10000 mm auf und der obere Verstärkungsteil der erfindungsgemäßen Umesterungskolonne einen Durchmesser D_{VT2} von 200 bis 10000 mm und eine Länge L_{VT2} von 200 bis 10000 mm auf. Das Verhältnis von Durchmesser des unteren Verstärkungsteils zu Kolonnendurchmesser in der Reaktionszone D_{VT1}:D_{RZ} liegt bevorzugt bei 0,3 bis 1 und das Verhältnis von Durchmesser des oberen Verstärkungsteils zu Kolonnendurchmesser in der Reaktionszone D_{VT2}:D_{RZ} bevorzugt bei 0,1 bis 1.

In weiteren bevorzugten Ausführungsformen weist der Zwischenkondensator der erfindungsgemäßen Umesterungskolonne eine Länge L_{C2} von 100 bis 10000 mm auf und das Verhältnis von Durchmesser des Zwischenkondensators zu Kolonnendurchmesser in der Reaktionszone D_{C2}:D_{Rz} liegt bevorzugt bei 0,1 bis 1. Weiterhin bevorzugt weist der Zwischenkondensator eine Wärmeübertragungsfläche A_{C2} von 1 bis 5000 m² auf.

Der Zwischenkondensator kann ist in die Kolonne integriert sein oder als separater Zwischenkondensator außerhalb der Umesterungskolonne ausgeführt sein. Des Weiteren können auch Zwischenkondensator und die obere Sektion des Verstärkungsteils nicht in die Umesterungskolonne integriert sondern separat außerhalb der Umesterungskolonne untergebracht sein. Für den Zwischenkondensator sind erfindungsgemäß verschiedene Konstruktionen möglich, wie z.B. Plattenwärmetauscher oder Rohrbündelwärmetauscher. Solche Konstruktionen sind dem Fachmann bekannt.

Die vorangehend aufgeführten Abmessungen und Kenndaten der erfindungsgemäßen Umesterungskolonne gelten vorzugsweise für eine Produkt-Menge von mehr als 100 kg/h, wobei unter Produkt-Menge gegebenenfalls die Summe des im Sumpfprodukt enthaltenen Alkylarylcarbonats und/oder Diarylcarbonates zu verstehen ist. Eine Umrechnung der gegebenen Abmessungen auf geringere Produktmengen ist dem Fachmann geläufig. Auch aus solchen Umrechnungen resultierende Umesterungskolonnen sind Gegenstand der vorliegenden Erfindung.

Für die erfindungsgemäße Kolonne gelten weiterhin die vorangehend für die erste Umesterungskolonne im erfindungsgemäßen Verfahren aufgeführten Vorzugsbereiche entsprechend.

Beispielhafte und zudem besonders bevorzugte Ausführungsformen der erfindungsgemäßen Kolonne sind den Fig. 1 bis 3 zu entnehmen.

Die folgenden Beispiele dienen der beispielhaften Erläuterung der Erfindung und sind nicht als Beschränkung aufzufassen.

### Beispiele

### Beispiel 1 (erfindungsgemäß)

In eine Umesterungskolonne aus
- einem oberen Verstärkungsteil (VT₂) mit 4 theoretischen Stufen
- einem Zwischenkondensator (C₂)
- einem unteren Verstärkungsteil (VT₁) mit 4 theoretischen Stufen
- einer Reaktionszone (RZ) mit 30 Reaktionsböden (Hold-up: 12 1), wobei 3 Böden mit Heizelementen ausgestattet sind und
- einem Abtriebsteil AT mit 6 Böden (Hold-up: 121)

werden 400 kg/h eines Gemisches aus 85.4 Gew.-% Phenol, 9.2 Gew.-% Dimethylcarbonat, 3.2 Gew.-% Diphenylcarbonat, 1.5 Gew.-% Titantetraphenolat, 0.3 Gew.-% Anisol, 0.3 Gew.-% Methylphenylcarbonat und 0.1 Gew.-% Methanol am oberen Ende der Reaktionszone zudosiert. Am unteren Ende der Reaktionszone werden 539,6 kg/h eines um 5 °C überhitzten Dampfgemisches aus 98.8 Gew.-% Dimethylcarbonat, 0.9 Gew.-% Phenol, 0.2 Gew.-% Anisol und 0.1 Gew.-% Methanol zugeführt.
Dabei erhält man am Sumpf der Kolonne 456.9 kg/h ein Produktgemisch bestehend aus 51 Gew.-% Phenol, 27.3 Gew.-% MPC (124.7 kg/h), 11.9 Gew.-% DPC (54.3 kg/h), 8.1 Gew.-% DMC, 0.4 Gew.-% Anisol und 1.3 Gew.-% Titantetraphenolat..
Die Umesterungskolonne wird bei einem Kopfdruck (oberhalb von VT₂) von 3.6 bar und einem Rücklaufverhältnis von 1.15 betrieben. Dabei wird im Sumpf der Kolonne eine Temperatur von 230 °C und in der Reaktionszone eine mittlere Reaktionstemperatur von 215 °C eingestellt. Ein Sumpfverdampfer E₁ und Zwischenverdampfer E₂ - E₄ in der Reaktionszone werden mit Heizdampf bei einem Dampfdruck von 35 bar betrieben, wobei als Sumpfverdampfer E₁ ein Naturumlaufverdampfer verwendet wird und als Zwischenverdampfer auf den Reaktionsböden integrierte Heizregister verwendet werden. Die Eintrittstemperatur in den Zwischenkondensator beträgt 205°C, die Austrittstemperatur 193 °C und die Kühlleistung 57 kW. Die bei der Zwischenkondensation anfallende Kondensationswärme kann zur Erzeugung von Heizdampf mit einem Heizdampfdruck von 8 bar (Tautemperatur: 170.4 °C) verwendet werden. Die erforderliche Heizleistung zur Verdampfung des Dimethylcarbonat enthaltenden Stromes beträgt 52 kW. Die Verdampfung und Überhitzung des Dimethylcarbonates erfolgt bei einer Temperatur von 135 bis 152 °C, wozu der im Zwischenkondensator verwendete Dampf problemlos verwendet werden kann.

Ohne die Verwendung des Zwischenkondensators C₂ und die Nutzung der dabei anfallenden Abwärme läge der Energieverbrauch der Umesterung einschließlich der Heizleistung der Verdampfer E₁ und E₂-E_{N} und der für die Verdampfung des Dimethylcarbonat enthaltenden Stromes bei 183 kW. Durch die Verwendung des Zwischenkondensators können bei der Umesterung 52 kW, also 28 % an Heizleistung eingespart werden. Berücksichtigt man zudem, dass im Zwischenkondensator 57 kW Wärme abgeführt werden, wird zudem ein Überschuss an Dampf (5 KW) erzeugt, der gegebenenfalls anderweitig genutzt werden kann. Die Kondensationswärme am Kopfkondensator (C1) beträgt 126 kW und erfolgt im Temperaturbereich 137 - 115 °C. Ohne Verwendung des Zwischenkondensators würde diese in etwa 183 kW betragen. Durch die Verwendung des Zwischenkondensators wird somit auch die Kühlleistung deutlich, d.h. um 31 % reduziert.

### Beispiel 2 (erfindungsgemäß)

In eine Kolonne aus
- einem oberen Verstärkungsteil (VT₂) mit 6 theoretischen Stufen
- einem Zwischenkondensator (C₂)
- einem unteren Verstärkungsteil (VT₁) mit 6 theoretischen Stufen
- einer Reaktionszone (RZ) mit 35 Reaktionsbödenböden (Hold-up: 12 1) ohne Zwischenverdampfer
- einem Abtriebsteil (AT) mit 9 Böden (Hold-up: 121)
werden 473.2 kg/h eines Gemisches aus 94.46 Gew.-% Phenol, 1.41 Gew.-% Dimethylcarbonat, 2.75 Gew.-% Diphenylcarbonat, 1.3 Gew.-% Titantetraphenolat, 0.01 Gew.-% Anisol, 0.02 Gew.-% Methylphenylcarbonat und 0.05 Gew.-% Methanol am oberen Ende der Reaktionszone zudosiert. Am unteren Ende der Reaktionszone werden 477.4 kg/h eines um 20 °C überhitzten Dampfgemisches bestehend aus 98,3 Gew.-% Dimethylcarbonat, 1.5 Gew.-% Phenol, 0.1 Gew.-% Anisol und 0.1 Gew.-% Methanol zugeführt.

Dabei erhält man am Sumpf der Kolonne 489.2 kg/h ein Produktgemisch bestehend aus 69.18 Gew.-% Phenol, 6.32 Gew.-% MPC (30.9 kg/h), 23.03 Gew.-% DPC (112.7 kg/h), 0.2 Gew.-% DMC, 0.01 Gew.-% Anisol und 1.26 Gew.-% Titantetraphenolat..

Die Umesterungskolonne wird bei einem Kopfdruck (oberhalb von VT₂) von 2 bar und einem Rücklaufverhältnis von 1.15 betrieben. Der Phenolgehalt im Destillat lag bei 2 Gew.-%. Dabei wird im Sumpf der Kolonne eine Temperatur von 227 °C und in der Reaktionszone eine mittlere Reaktionstemperatur von 200 °C eingestellt. Ein Sumpfverdampfer E₁ wird mit Heizdampf bei einem Dampfdruck von 35 bar betrieben, wobei als Sumpfverdampfer ein Naturumlaufverdampfer verwendet wird.

Die Eintrittstemperatur in den Zwischenkondensator beträgt 190°C, die Austrittstemperatur 183 °C und die Kühlleistung 46 kW. Die bei der Zwischenkondensation anfallende Kondensationswärme kann zur Erzeugung von Heizdampf mit einem Heizdampfdruck von 8 bar (Tautemperatur: 170.4 °C) verwendet werden. Die Verdampfung und Überhitzung des Dimethylcarbonates erfolgt bei einer Temperatur von 122 bis 153 °C, wozu der im Zwischenkondensator verwendete Dampf problemlos verwendet werden kann. Die erforderliche Heizleistung zur Verdampfung des Dimethylcarbonat enthaltenden Stromes beträgt 46 kW.

Ohne die Verwendung des Zwischenkondensators C2 und die Nutzung der dabei anfallenden Abwärme läge der Energieverbrauch der Umesterung einschließlich der Heizleistung der Verdampfer E₁ und E₂-E_{N} und der für die Verdampfung des Dimethylcarbonat haltigen Stromes bei 190 kW. Durch die Verwendung des Zwischenkondensators können bei der Umesterung 46 kW, also 24 %, an Heizleistung eingespart werden. Die Kondensationswärme am Kopfkondensator (C1) beträgt 144 kW und erfolgt im Temperaturbereich 137 - 115 °C. Ohne Verwendung des Zwischenkondensators würde diese 190 kW (siehe Vergleichsbeispiel 3) betragen. Durch die Verwendung des Zwischenkondensators wird somit auch die Kühleistung deutlich, d.h. um 24 % reduziert.

### Vergleichsbeispiel 3

Unter sonst gleichen Bedingungen wie in Beispiel 2 wurde die Umesterungskolonne ohne Zwischenkondensator betrieben. Zur Erreichung eines Phenolgehaltes von 2 Gew.-% im Destillat musste das Rücklaufverhältnis auf 2 angehoben werden.
Die Kühlleistung des Kopfkondensators C1 betrug 190 kW, die des Verdampfers 144 kW. Die Kondensation im Kopfkondensator C1 erfolgte bei Temperaturen zwischen 125 und 97 °C.

Aufgrund der verhältnismäßig niedrigen Temperaturniveaus bei der Kondensation im Kopfkondensator, ist die anfallende Kondensationswärme für viele Prozesse nicht mehr nutzbar und muss daher letztendlich mittels Kühlwasser oder Luftkühlung abgeführt werden.

Erfindungsgemäß wird daher mittels Ausführung mit Zwischenkondensator dem Stoffstrom in der Kolonne Energie auf einem höheren Temperaturniveau als am Kopfkondensator entnommen, so dass die Energie für die Verdampfung eines der Kolonne zugeführten Stromes eingesetzt werden kann. Damit ist eine Einsparung erheblicher Energiemengen verbunden.

## Patentansprüche

1. Verfahren zur Herstellung von wenigstens einem Diarylcarbonat und/oder Alkylarylcarbonat aus wenigstens einem Dialkylcarbonat und wenigstens einer aromatischen Hydroxyverbindung, wobei
(a) das oder die Dialkylcarbonat(e) in Gegenwart wenigstens eines Umesterungskatalysators mit der oder den aromatischen Hydroxyverbindung(en) in wenigstens einer Umesterungskolonne enthaltend wenigstens einen Verstärkungsteil im oberen Teil der Kolonne und wenigstens eine Reaktionszone unterhalb des Verstärkungsteils, welches mindestens zwei Sektionen aufweist, umgesetzt werden,
(b) der am Kopf der Umesterungskolonne entnommene Dampf in wenigstens einem Kondensator ganz oder teilweise kondensiert wird,
**dadurch gekennzeichnet, dass** wenigstens ein Verstärkungsteil der Umesterungskolonne mit wenigstens einem Zwischenkondensator ausgestattet ist und die durch Kondensation in diesem Zwischenkondensator gewonnene Kondensationswärme direkt oder indirekt wieder in das Verfahren zurückgeführt wird.

2. Verfahren gemäß Anspruch 1, **dadurch gekennzeichnet, dass** die Umesterungskolonne unterhalb einer Reaktionszone wenigstens einen Abtriebsteil aufweist.

3. Verfahren gemäß Anspruch 1 oder 2, **dadurch gekennzeichnet, dass** das bei der Kondensation des am Kopf der Umesterungskolonne entnommenen Dampfes anfallende Kondensat teilweise wieder als Rücklauf auf die Umesterungskolonne aufgegeben wird.

4. Verfahren gemäß wenigstens einem der Ansprüche 1 bis 3, **dadurch gekennzeichnet, dass** die gewonnene Kondensationswärme direkt oder indirekt, ganz oder teilweise zur Verdampfung des in die Umesterungskolonne eingeleiteten Dialkylcarbonats verwendet wird.

5. Verfahren gemäß wenigstens einem der Ansprüche 1 bis 4, **dadurch gekennzeichnet, dass** der Zwischenkondensator in die Umesterungskolonne integriert oder als separater Zwischenkondensator außerhalb der Kolonne ausgeführt ist.

6. Verfahren gemäß wenigstens einem der Ansprüche 1 bis 5, **dadurch gekennzeichnet, dass** der Verstärkungsteil der Umesterungskolonne, der mit wenigstens einem Zwischenkondensator ausgestattet ist, in einen unteren und einen oberen Verstärkungsteil unterteilt ist, wovon sich der untere Verstärkungsteil unterhalb des Zwischenkondensators und der obere Verstärkungsteil oberhalb des Zwischenkondensators befindet.

7. Verfahren gemäß Anspruch 6, **dadurch gekennzeichnet, dass** der Zwischenkondensator und der obere Verstärkungsteil in die Umesterungskolonne integriert oder als separate Vorrichtung außerhalb der Kolonne ausgeführt sind.

8. Verfahren gemäß wenigstens einem der Ansprüche 1 bis 7, **dadurch gekennzeichnet, dass** die Temperatur der Reaktionszone im Bereich von 100 bis 300°C, bevorzugt von 120 bis 250°C, besonders bevorzugt von 150 bis 240°C und der Druck der Reaktionszone im Bereich von 0.5 bis 20 bar, bevorzugt von 0.8 bis 15 bar, besonders bevorzugt von 0.9 bis 10 bar liegen.

9. Verfahren gemäß wenigstens einem der Ansprüche 1 bis 8, **dadurch gekennzeichnet, dass** es sich bei dem Diarycarbonat um Diphenylcarbonat, bei dem Dialkylcarbonat um Dimethylcarbonat oder Diethylcarbonat und bei der aromatischen Hydroxyverbindung um Phenol handelt.

10. Kolonne für die Umsetzung von Dialkylcarbonaten mit aromatischen Hydroxyverbindungen in Gegenwart wenigstens eines Umesterungskatalysators zur Herstellung von Diarylcarbonaten und/oder Alkylarylcarbonaten enthaltend
• wenigstens einen Einlass für das oder die Dialkylcarbonat(e) und wenigstens einen Einlass für das oder die Hydroxyverbindung(en)
• wenigstens einen Auslass für das gasförmige Kopfprodukt im oberen Teil der Kolonne und wenigstens einen Auslass für das flüssige Sumpfprodukt im unteren Teil der Kolonne
• wenigstens einen Verstärkungsteil im oberen Teil der Kolonne und wenigstens eine Reaktionszone unterhalb eines Verstärkungsteils, welcher mindestens zwei Sektionen aufweist.
**dadurch gekennzeichnet, dass** wenigstens ein Verstärkungsteil mit wenigstens einem Zwischenkondensator ausgestattet ist.

11. Kolonne gemäß Anspruch 10, **dadurch gekennzeichnet, dass** sie unterhalb einer Reaktionszone wenigstens einen Abtriebsteil aufweist.

12. Kolonne gemäß Anspruch 10 oder 11, **dadurch gekennzeichnet, dass** der Verstärkungsteil, der mit wenigstens einem Zwischenkondensator ausgestattet ist, in einen unteren und einen oberen Verstärkungsteil unterteilt ist, wovon sich der untere Verstärkungsteil unterhalb des Zwischenkondensators und der obere Verstärkungsteil oberhalb des Zwischenkondensators befindet.

13. Kolonne gemäß wenigstens einem der Ansprüche 10 bis 12, **dadurch gekennzeichnet, dass** der Zwischenkondensator und gegebenenfalls der obere Verstärkungsteil in die Kolonne integriert oder als separate Vorrichtung außerhalb der Kolonne ausgeführt sind.
